# EUROPEAN PATENT APPLICATION

(11) **EP 2 578 697 A1**
(43) Date of publication of application: **10.04.2013**
(21) Application number: 11184319.9
(22) Date of filing: 07.10.2011
(51) Int. Cl.: C12Q 1/68

(54) **Estimation of drug-metabolizing capacity**

(71) Applicant: TOXI-COOP Toxicological Research Center Zrt, 1124 Budapest (HU); Chemical Research Center Hungarian Academy Of Science, 1025 Budapest (HU); Paulik, József, 1038 Budapest (HU)
(72) Inventor: Monostory, Katalin, 1124 Budapest (HU); Kóbori, László, 1141 Budapest (HU); Paulik, József, 1038 Budapest (HU)
(74) Representative: Lengyel, Zsolt

(57) **Abstract**

The present invention provides a method for the estimation of a patient's drug-metabolizing capacity by parallel CYP-genotyping and CYP-phenotyping, comprising identification of specific mutation(s) within the gene encoding said CYP enzyme; determination of the expression level of the gene encoding said CYP enzyme; and estimation of the drug-metabolizing capacity of said patient on the basis of CYP-genotyping data and pre-established cut-off values of said expression level, thereby qualifying said patient as poor, intermediate or extensive metabolizer.

## Description

The present invention provides a method for the estimation of a patient's drug-metabolizing capacity by parallel CYP (cytochrome P450)-genotyping and CYP-phenotyping, comprising identification of specific mutation(s) within the gene encoding said CYP enzyme; determination of the expression level of the gene encoding said CYP enzyme; and estimation of the drug-metabolizing capacity of said patient on the basis of CYP-genotyping data and pre-established cut-off values of said expression level, thereby qualifying said patient as poor, intermediate or extensive metabolizer.

The lack of therapeutic effect of a drug or the appearance of undesired side-effects, resulting in adverse events for patients, is partly caused by differences or changes in drug-metabolism. Significant importance in inter-individual differences is of genetic variability (polymorphism) of drug-metabolizing enzymes, causing reduced or even no enzyme activity. As an effect, drug-metabolizing capacity of a patient can be weaker ('poor metabolizer') compared to the other members ('intermediate or extensive metabolizer') of population (Ingelman-Sundberg, 2001). An individual with poor drug-metabolism can live normal life, until he/she is treated with a drug metabolized by an enzyme with reduced or no activity. Patients with poor drug-metabolizing capacity can produce significantly higher blood levels of certain drugs, causing more severe and frequent side-effects (Brockmöller et al., 2000; Wilke et al., 2005).

The principal organ of drug-metabolism is the liver; however, every tissue has some ability to metabolize xenobiotics. Although drug-metabolizing activities of gut wall, kidneys, lungs or even brain can contribute to the overall biotransformation to some extent, the drug-metabolizing capacity of patients can be roughly estimated by hepatic metabolism. Therapeutic failure or drug toxicity is highly influenced by hepatic drug-metabolizing capacity, primarily depending on the levels and activities of CYP enzymes. The enzymes belonging to CYP1-3 families play a central role in biotransformation of various drugs to more polar compounds, which are readily excreted (Lewis, 2004; Monostory and Pascussi, 2008). One of the most important reasons for inter-individual variation in drug-metabolism is genetic polymorphism of *CYP* genes. Some *CYP* genes (*CYP2C9, CYP2C19, CYP2D6, CYP3A5)* are highly polymorphic, resulting in enzyme variants with reduced or even no activity (Solus et al., 2004). The genetically determined variance in CYP enzyme activities is temporarily modulated by environmental (nutrition, co-medication) or internal factors (age, hormonal status, liver function, diseases), leading to different drug-metabolism phenotypes (Monostory and Pascussi, 2008). Individuals with defective *CYP* alleles display permanent poor drug-metabolism, whereas those who have wild type *CYP* genes may become temporary poor metabolizers. It means that CYP-phenotype and drug-metabolizing capacity dynamically changes in the course of external and internal influences, accommodating to everyday chemical exposure. A temporary weak drug-metabolizing state can arise due to a run-down of physical and health condition, and consumption of certain drugs or citruses. On contrary, an ultra-fast metabolism can occur upon St. John's wort tea consumption, or during treatment with steroids or rifampicin (Monostory and Pascussi, 2008).

Recognizing individual differences in drug-efficiency and toxicity, personalized drug therapy adjusted to patients' drug-metabolizing capacity can promote to avoid potential side-effects of drugs. Tailored medication as a part of modem medical practices requires establishing reliable diagnostic background for identification of inactivating mutations or lack of functional CYP-activities. In the past years, pharmacogenetic services for the estimation of drug-metabolizing capacity have risen worldwide (Brockmöller et al., 2000; Wilke et al., 2005); however, the test services present in the market offer to determine non-functional CYP enzymes only by CYP-genotyping, and do not provide information about drug-metabolizing capacity of patients who do not have CYPmutations. These test services focus on CYP-genotyping analysis for clinically relevant mutations in *CYP* genes which determines the permanent poor metabolism, since the defective *CYP* alleles produce enzymes with reduced activity or even non-functional enzymes.

CYP-phenotyping analysis can provide information on the current hepatic CYP activities and can predict temporary poor metabolism in those subjects who do not carry mutations in *CYP* genes. Drug-metabolism in the liver can be roughly estimated by the activities of the most relevant drug-metabolizing CYP enzymes; however, the assays of CYP enzyme activities require a large amount of liver tissues. It can be considered to be a significant drawback in testing drug-metabolizing capacity from liver needle-biopsies, where the available tissue is limited. In addition, liver biopsies are generally not available from patients, since it is risky and impractical to obtain specimens from the liver in patients. It should be mentioned that substantially lower CYP enzyme activities can be measured in some extrahepatic tissues, such as kidneys; however the availability of those tissues is as limited as for the liver.

The levels of mRNAs as the prerequisite for enzyme proteins and enzyme activities can be determined in small amounts of biological material. Real-time PCR techniques for measuring CYP expressions provide a useful method for characterization of liver drug-metabolizing capacity. For CYP expression studies, the peripheral blood was investigated as an obvious choice for easily accessible type of tissues, presumably reflecting drug-metabolizing capacity of the liver (CYP enzyme activities are practically undetectable in the blood, thus CYP enzyme activities in blood cells are not expected to reflect the liver activities, and thus blood is not useful tissue for CYP enzyme activity assays). The mature human erythrocytes, the main cellular components of blood are anucleate cells, thus, they are not capable of active RNA synthesis. However, there is strong evidence that red blood cells have substantial RNA content (Kabanova et al. 2009). This fact supports the assumption of nucleus independent protein synthesis, and the obvious lack of the transcriptional regulation of gene expression in mature erythrocytes. Consequently, mRNA levels of various genes indicate the current regulatory effects of environmental and internal factors before the moment of nucleus discarding, and do not display prompt transcriptional response on temporary modulation during the 120-day lifespan in circulation. On the other hand, leukocytes can also be chosen as the target cells of CYP expression studies, which are nucleated and display active RNA synthesis. Although several efforts have been undertaken to establish CYP mRNA levels in liver tissues and in blood or peripheral blood mononuclear cells (Finnström et al., 2001 and 2002; Nowakowski et al., 2002; Koch et al., 2002; Lind et al., 2003; Furukawa et al., 2004; Lee et al., 2010), the currently available data on CYP expressions in blood cells were not sufficient for estimation of hepatic CYP activities and hepatic drug-metabolizing capacity, thus a comprehensive analysis remains elusive.

It can be seen from the above discussion of the state of the art that the currently available methodologies are in need of substantial improvements to provide better tools for estimation of drug-metabolizing capacity of a patient to allow improved treatment options. The present inventors accomplish this goal, providing a global approach by combining CYP-genotyping and CYP-phenotyping tools for estimation of patients' drug-metabolizing capacity. This approach can add novel elements to the available diagnostics, and combination of CYP-genotyping and CYP-phenotyping enables more accurate qualification of patients' drug-metabolism. In contrary to the already existing assays, this multi-step diagnostic system qualifies the patient's current drug-metabolizing capacity, and suggests more rational drug therapy adjusted to the results. The complex diagnostic system provides an opportunity to predict the possible CYP enzyme defects or extremely reduced/increased CYP expression that identifies the limitations of drug therapy. By estimation of patient's drug-metabolizing capacity, modification of drug therapy in a rational, individually adjusted way can lower the incidences of adverse drug reactions. The quality of patients' life can eventually be improved, if the shortness of drug-metabolizing capacity is recognized on time, and an individually adjusted therapy is applied.

During the development of the present diagnostic system, a further surprising finding strengthened the robustness of the technology: a correlation between the expressions of several drug-metabolizing CYP enzymes in the liver and in leukocytes (peripheral blood) was established. The ability to utilize an easily accessible patient's sample provides significant additional advantages to the system according of the present invention. If CYP-status of leukocytes can provide valuable information on the drug-metabolizing capacity of the liver, the qualification of patients' drug-metabolizing capacity will have predictive power regarding forthcoming medication. Prospective investigation of CYP-status allows prediction of potential 'poor (or ultra-rapid) metabolizer' phenotypes and facilitates improvement of the individual therapy, leading to the optimization of drug choice and/or dose for a more effective therapy, to avoid serious adverse effects, and to decrease medical costs.

Accordingly, the present invention provides a method for the estimation of a patient's drug-metabolizing capacity by parallel CYP-genotyping and CYP-phenotyping of said patient, comprising
a) identification of specific mutation(s) within the gene encoding said CYP enzyme from a leukocyte sample obtained from said patient,
b) determination of the expression level of the gene encoding said CYP enzyme from said leukocyte sample,
c) estimation of the drug-metabolizing capacity of said patient on the basis of CYP-genotyping data and pre-established cut-off values of said expression level determined from said leukocyte sample, thereby qualifying said patient as poor, intermediate or extensive metabolizer.

In a preferred embodiment, the method according to the invention is used in connection with any of the CYP enzymes from the group consisting of CYP1A2, CYP2C9, CYP2C19 and/or CYP3A4.

In another preferred embodiment, said determination of specific mutations within the gene encoding said CYP enzyme is made by single nucleotide polymorphism analysis.

In a further preferred embodiment, said determination of the expression level of the gene encoding said CYP enzyme is made by real time-PCR analysis.

### Detailed description

Personalized medication of modem therapy highly requires reliable diagnostic tools for the estimation of patients' current drug-metabolizing capacity. Although the assessment of overall drug-metabolism can be hardly accomplished, much information can be obtained by some simplification: i) the enzymes concerned in the biotransformation of drugs are primarily located in liver; ii) the majority of drugs are metabolized by CYP enzymes; iii) the metabolism of about 90% of drugs in clinical use is catalyzed by the enzymes belonging to the CYP1-3 family (e.g. CYP1A2, CYP2B6, CYP2C9, CYP2C19, CYP2D6 and CYP3As) (Chen et al., 2011). Thus, the drug-metabolizing capacity can be roughly assessed through integrative analysis of the current expression (mRNA levels and activities) of these CYP enzymes in the liver and/or leukocytes and the genomic identification of defective *CYP* alleles. The qualification of patient's drug-metabolizing status together with personalized medication can be utilized for the improvement of drug therapy, resulting in increased drug efficacy and decreased risk of adverse drug events.

When leukocytes are chosen as the target cells of CYP status assays, a series of questions arises: i) does the expression of drug-metabolizing CYP genes in leukocytes reflect the hepatic CYP activities; ii) is the regulation of CYP expression in leukocytes similar to that of in liver; iii) can we obtain information on hepatic drug-metabolizing capacity from leukocytes. In order to answer these questions, the present study was designed to investigate CYP mRNA levels in leukocytes collected simultaneously with liver tissues, and to study the correlation between CYP expression and hepatic CYP activities.

As outlined above, the present invention therefore provides a method for estimation of patient's drug-metabolizing capacity by parallel CYP-genotyping and CYP-phenotyping. For obtaining meaningful data for correlation analysis, a baseline study was performed to establish the CYP enzyme activities in liver. The basic methods for the estimation of drug-metabolizing capacity are determination of catalytic activities of individual CYPs by CYP-selective activity probes (Yuan et al., 2002). Although these techniques are reliable in hepatic microsomes, the catalytic analyses require a relatively large amount of liver tissues, which is a serious drawback for human studies. CYP enzyme activities are practically undetectable in leukocytes, thus the correlation between liver and blood activities cannot be analyzed. The enzyme activities of the six most relevant drug-metabolizing CYPs (CYP1A2, CYP2B6, CYP2C9, CYP2C19, CYP2D6 and CYP3A4) were determined in liver tissues of cadaveric donors. The hepatic CYP activities of different donors showed a wide variation as it was reported by several authors (Transon et al., 1996; Blanco et al., 2000; Shu et al., 2001). Inter-individual variability in CYP activities in liver microsomes ranged from 80- to 750-fold variation for CYP2B6 and CYP3A4, respectively, or from non-detectable to rather high values for CYP1A2, CYP2C9, CYP2C19 and CYP2D6.

The method of the invention may use different types of biological samples. As detailed above, the liver sample, if available, could provide an obvious choice for the isolation of genomic DNA and total RNA, and for further analysis of CYP status. As also mentioned, the most widely available source is the blood sample, containing sufficient amount of genomic DNA and total RNA, can also be used for CYP-genotyping and CYP-fenotyping. Intact or analyzable nucleic acids are readily extracted from several various types of biological samples, such as saliva and other sources routinely used for genetic testing.

In the method of the invention, the expression levels of the genes encoding the CYP enzymes are to be determined. Methods for this determination are widely available, and are well known for the person skilled in the art. These include for example real time-PCR analysis. The measurements for different CYP expression levels from the same sample can be simultaneous (e.g. microarray arrangement), or the determinations may be done separately (manually or on automated equipment).

For establishing CYP mRNA expression, the samples used for enzyme activity testing were also assayed for CYP expression by the real time PCR method. Variations in hepatic CYP mRNAs were more or less similar to the variations in CYP activities, ranging from 20- to 2000-fold for CYP2D6 and CYP3A4, respectively. The liver tissues of all donors contained detectable amounts of various CYP mRNAs in contrast to the hepatic activities which were non-detectable in some cases. The expression of all *CYP* genes investigated was also detectable in leukocytes, contrary to those observed in other studies. Koch et al. (2002) could not detect mRNAs of CYP3A subfamily, whereas Furukawa et al. (2004) could not display the expression of CYP2C9, CYP2C19, CYP2D6 and CYP3A4 in peripheral blood cells at all. Artefactual causes due to inappropriate sample collection and storage or methodological problems caused by inadequate primer/probe design might have led to their negative results. In our study, the relative expression of various *CYP* genes were generally lower in leukocytes than in liver tissues (about 20- to 3*10⁴-fold lower), and the variations in CYP mRNAs in leukocyte were higher than in liver, ranging from 60- to 10⁵-fold variations.

The relationship between hepatic mRNA levels and CYP activities were established to find evidence for that the expression of *CYP* genes could provide accurate information on respective CYP enzyme activities. Strong correlation (rₛ>0.87) was displayed between CYP1A2 mRNA and phenacetin O-dealkylation. This fact suggests that the hepatic expression of *CYP1A2* gene reflects to CYP1A2 activities in liver. Similar conclusion was drawn from the results of Rodriguez-Antona et al. (2001) or of George et al. (1995), obtaining potential association between the hepatic CYP1A2 mRNA levels and 7-methoxyresorufin O-demethylation activity or CYP1A2 protein content, respectively. In our study with 164 liver donors, we also found strong relationship between hepatic mRNA levels and mephenytoin N-demethylation of CYP2B6 (rₛ=0.89). However, Rodriguez-Antona et al. (2001) reported weaker correlation between hepatic CYP2B6 mRNA levels and benzoxyresorufin O-debenzylation activities of 12 human liver samples (r=0.52). The fact that benzoxyresorufin O-debenzylation is catalyzed not only by CYP2B6, but also by CYP3A4 (Niwa et al., 2003) could account for the lower correlation coefficient found in CYP2B6 by Rodriguez-Antona et al. (2001).

The present study found that CYP2C9, CYP2C19, CYP2D6 and CYP3A4 showed somewhat weaker correlation, if hepatic mRNA levels and activities were compared for all donors. Genetic polymorphisms, producing less active CYP enzymes or even null activities could be responsible for the fact that some liver tissues displayed rather high mRNA levels, but low activities of CYP2C9, CYP2C19, or CYP2D6.

Accordingly, the method of the invention includes a step for the determination the presence and/or absence of specific mutation(s) within the genes encoding the CYP enzymes from a biological sample. The methods for this determination are widely available, and are well known for the person skilled in the art. These include for example single nucleotide polymorphism (SNP) analysis by high resolution melting curve analysis or by hydrolysis SNP analysis, and even complete gene sequencing. The analysis of the different *CYP* gene sequences from the same sample can be simultaneous (e.g. microarray arrangement), or the determinations may be done separately (manually or on automated equipment).

At present, more than 100 allelic variants of CYP families 1-3 are displayed in human populations; however, measuring all polymorphisms would not be cost effective. While there are many allelic variants for some CYP enzymes, some variants are rare in general and others vary substantially in prevalence by ethnic groups. Table 1 lists the frequencies of some *CYP* allelic variants in major ethnic groups, showing that one or few alleles with relatively high prevalence in one group may be rare in another.

**Table 1: Frequencies of CYP allelic variants with clinically relevant consequences**

| **CYP allele** | **Consequence** | **Caucasian** | **Frequency (%) Black African / Afro-American** | **Asian** |
|---|---|---|---|---|
| CYP2C9*2 | Reduced enzyme activity | 8-14 | 1 | 0 |
| CYP2C9*3 | Reduced enzyme activity | 4-16 | 1-2 | 2-3 |
| CYP2C19*2 | Inactive | 15 | 17 | 25-30 |
| CYP2C19*3 | Inactive | 0.04 | 0.4 | 8 |
| CYP2D6*3 | Inactive | 2 | 0 | 0 |
| CYP2D6*4 | Inactive | 12-21 | 2-4 | 1 |
| CYP2D6*5 | No enzyme | 2-7 | 4 | 6 |
| CYP2D6*6 | Inactive | 1 | | |
| CYP2D6*10 | Reduced enzyme activity | 1-2 | 6 | 51 |
| CYP2D6*17 | Reduced enzyme activity | 0 | 34 | 0 |
| CYP2D6duplication | Ultra-rapid activity | 1-5 | 2 | 0-2 |
| CYP3A5*3 | No enzyme | 90-93 | 32 | 60-73 |
| CYP3A5*6 | | 0 | 12-13 | 0 |
| CYP3A5*7 | | 0 | 8-10 | 0 |

Thus, variants with serious clinical consequences and with relatively high frequency in the given population should be routinely measured. For instance, *CYP2C9*2, CYP2C9*3, CYP2C19*2, CYP2D6*4* and CYP3A5*3 should be included in CYP-genotyping assays if Caucasian populations are studied, *CYP2C19*2, CYP2D6*17*, *CYP3A5*3*, *CYP3A5*6* and *CYP3A5*7* are necessary to be included, if black African or Afro-American populations are studied, while *CYP2C19*2*, *CYP2C19*3*, *CYP2D6*10* and *CYP3A5*3* should be included in genotyping tests if Asians are studied. The person skilled in the art will be readily able to apply these modifications to the method of the invention according to the source of the samples to be analyzed.

In this step of our investigation, a preliminary CYP-genotyping study, detecting the clinically most relevant polymorphisms for these genes was carried out before the estimation of the relationship between hepatic CYP mRNA levels and activities. The donors carrying mutant *CYP* alleles (heterozygous or homozygous for *CYP2C9*2*, *CYP2C9*3*, *CYP2C19*2*, *CYP2C19*3*, *CYP2D6*3*, *CYP2D6*4*, *CYP2D6*6*) were excluded from correlation analyses. The high correlation coefficients (rₛ>0.88) indicated that the estimation of hepatic expression of *CYP2C9*, *CYP2C19* and *CYP2D6* genes instead of the respective CYP activities is appropriate. George et al. (1995) also reported significant, but not close correlation between CYP2C9 mRNA and enzyme protein which was presumably due to the poor selectivity of mRNA probe and CYP2C antibody, recognizing not only CYP2C9, but several other CYP2Cs. Rodriguez-Antona et al. (2001) described the lack of correlation between CYP2C9 mRNA levels and diclofenac 4'-hydroxylation; however, they did not take allelic variants producing inactive enzymes into account. They found lower correlation between the hepatic CYP2D6 mRNA levels and dextromethorphan O-demethylation than we displayed, most likely because of neglecting *CYP2D6* polymorphic alleles.

For CYP3A, liver tissues of some donors showed relatively high activities (nifedipine oxidation, midazolam 1'- and 4-hydroxylation), but low CYP3A4 expression. CYP3A5 polymorphism was assumed to be due to the weak correlation, since the functional CYP3A5 enzyme as the product of *CYP3A5*1* allele is also able to catalyze metabolism of CYP3A4 substrates to some extent. The relatively rare *CYP3A5*1* allele (about 5-10% of Caucasian population) and eventually the functional CYP3A5 enzyme can contribute to the overall metabolism of CYP3A substrates, such as nifedipine, midazolam, cyclosporine, tacrolimus, erythromycin, carbamazepine, or lidocaine (Patki et al., 2003; Dai et al., 2006; Huang et al., 2004). Thus, we excluded the donors with *CYP3A5*1* allele from the correlation analysis which resulted in much stronger correlation between the hepatic CYP3A4 mRNAs and CYP3A activities (rₛ>0.8 for nifedipine oxidation, midazolam 1'- and 4-hydroxylation). In previous studies by Sumida et al. (1999) and by Rodriguez-Antona et al. (2001), a rather high correlation between CYP3A4 mRNA amounts and testosterone 6β-hydroxylation was observed. The CYP3A4 expression in liver also seemed to be related to normalized plasma concentrations (plasma level/dose***weight) of the CYP3 A substrates, cyclosporine or tacrolimus in liver transplants (Thörn et al., 2004).

CYP expression levels in leukocytes may provide a tool to estimate drug-metabolizing capacity of the liver; however, information about intra-individual correlation between hepatic CYP activities and blood CYP gene expression is virtually non-existent. Finnström et al. (2001) demonstrated no relationship between mRNA levels of CYP1A2 and CYP3A4 in blood and in liver which might have been due to the differences in maturation processes and turnover rates of various subsets of blood cells, and due to the inclusion of all patients irrespective of their CYP-genotypes. No association between the expression of CYP1A2 in liver and in leukocytes was observed by Furukawa et al. (2004); furthermore, CYP2C9, CYP2C19 or CYP3A4 mRNAs were undetectable in leukocytes. The yield rate of RNA from leukocytes, inappropriate sample storage or less sensitive analytical method can be the limitations for detection of CYP mRNAs in leukocytes. Lee et al. (2010) reported poor correlation of CYP3A4 mRNA levels between liver and leukocytes, although the limited samples (n=5) might not provide strong evidence for correlation. We demonstrated here that mRNA levels of CYP1A2, CYP2C9, CYP2C19 and CYP3A4 in leukocytes highly correlated with the respective CYP activities in liver, allowing a good estimation of hepatic drug-metabolizing activities of these CYPs. The preliminary CYP-genotyping for clinically relevant mutations in *CYP* genes was necessary to discard the subjects carrying polymorphic *CYP* alleles which are generally transcribed, but are not associated with enzyme activities. The leukocyte CYP expression of the donors with homozygous wild genotypes for *CYP2C9* and *CYP2C19,* and with homozygous mutant genotypes for *CYP3A5* highly reflected to the hepatic activities of CYP2C9, CYP2C19 and CYP3A. It should be noted that mRNA levels of CYP2B6 and CYP2D6 in leukocytes did not show any relationship to the respective CYP activities in the liver. Although polymorphic *CYP2D6* alleles can provide information on CYP2D6 poor metabolism, the current CYP2D6 activities in liver cannot be estimated from CYP2D6 mRNA levels in leukocytes.

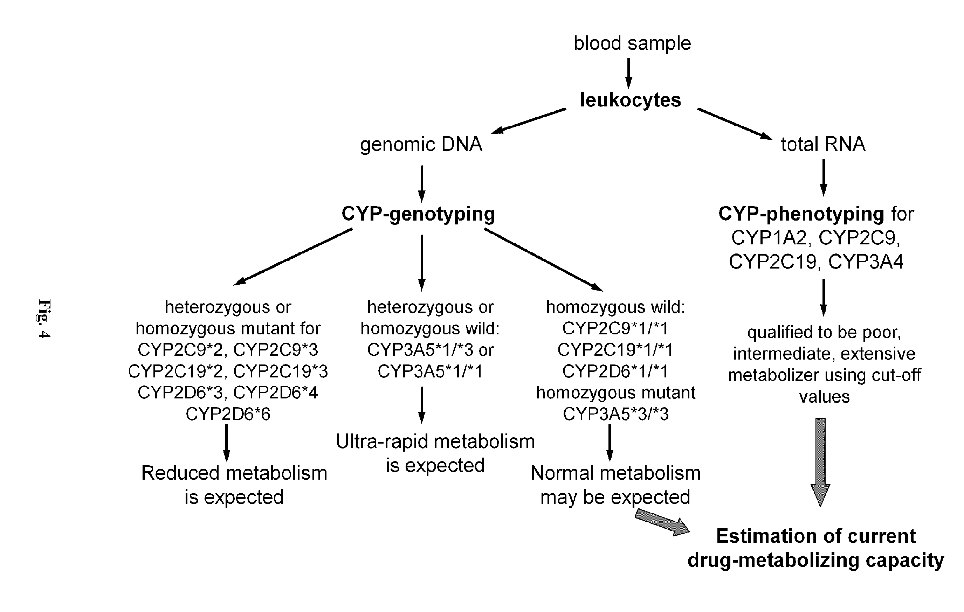

Accordingly, hepatic CYP activities can be estimated by combining CYP-genotyping and CYP-phenotyping analysis of liver biopsy samples or leukocytes. CYP-genotyping analysis for clinically relevant mutations in *CYP* genes (*CYP2C9*2*, *CYP2C9*3*, *CYP2C19*2*, *CYP2C19*3*, *CYP2D6*3*, *CYP2D6*4*, *CYP2D6*6* and *CYP3A5*3*) is suggested to carry out first. CYP-genotyping determines the permanent poor metabolism (or ultra-rapid metabolism), since the defective *CYP* alleles produce enzymes with reduced activity or even non-functional enzymes. CYP-phenotyping analysis of liver samples for CYP1A2, CYP2B6, CYP2C9, CYP2C19, CYP2D6 and CYP3A4 provides information on the current hepatic CYP activities in those subjects who do not carry mutations in *CYP2C9*, *CYP2C19* and *CYP2D6* genes and have *CYP3A5*3*/**3* genotype. Leukocytes from peripheral blood are more easily accessible biological samples, therefore CYP-phenotyping analysis of leukocytes can be preferred to the analysis of liver samples. CYP-phenotyping for CYP1A2, CYP2C9, CYP2C19 and CYP3A4 in leukocytes provides information on the current hepatic CYP activities in those subjects who carry homozygous wild genotypes of *CYP2C9* and *CYP2C19* or homozygous mutant genotype of *CYP3A5* (Fig. 4). In conclusion, a patient's drug-metabolizing capacity can be qualified by CYP-genotyping and CYP-phenotyping in liver samples and also in leukocytes, using the pre-established cut-off values of the expression level determined for the sample used, thereby qualifying said patient as poor, intermediate or extensive metabolizer. For this purpose, the present study provides said cut-off values based on a large sample size of 164 donors. The person skilled in the art will be able to readily apply these cut-off values when analysing an actual sample for its drug metabolizing capacity.

The profile of patients' genetic and non-genetic variations in drug-metabolism can guide the selection of drugs and optimal dose that can minimize the harmful side effects and ensure a more successful outcome. Tailored medication will eventually contribute to the improvement of quality of patients' lives.

### Brief description of the figures

- **Fig. 1.**: Frequency distribution of CYP1A2, CYP2B6, CYP2C9, CYP2C19, CYP2D6 and CYP3A activities in Hungarian liver donors (n=164).
- **Fig. 2.**: Correlation of relative *CYP* gene expression and corresponding CYP activities in liver. If Spearman's correlation coefficient (rₛ) was higher than 0.7, CYP expression levels and the corresponding hepatic CYP activities were defined as closely associated.
- **Fig. 3.**: Correlation of relative *CYP* gene expression in leukocytes and corresponding CYP activities in liver. If Spearman's correlation coefficient (rₛ) was higher than 0.7, CYP expression levels in leukocytes and the corresponding hepatic CYP activities were defined as closely associated.
- **Fig. 4.**: Estimation procedure of a patient's drug-metabolizing capacity in leukocytes isolated from peripheral blood.

The present invention is further illustrated by the experimental examples described below; however, the scope of the invention will by no means be limited to the specific embodiments described in the examples.

### Example 1: Materials and methods

**Liver and blood samples.** The drug-metabolizing capacity of human liver samples not selected for transplantation (n=146) or liver tissues remaining after reduced-size transplantation (n=18) was determined by CYP-genotyping and CYP-phenotyping approaches. In parallel, samples of peripheral blood were also taken from all donors. The male/female ratio of the donors was 91/73, their age ranged between 3 and 74 years (43.4±14.46 years), and the cause of death included intracranial bleeding (65.2%) and cerebral contusion (34.8%). The livers were retrieved from hemodynamically stable brain death donors with normal liver function. The livers were perfused and stored in HTK (Fresenius AG, Bad Homburg v.d.H., Germany). The use of human tissues for scientific research was approved by the Hungarian Committee of Science and Research Ethics. All experimental activities were carried out under the regulation of Act CLIV of 1997 on Health and the decree 23/2002 of the Minister of Health of Hungary.

Microsomes and total RNA were isolated from the liver samples. Liver tissues were homogenized in 0.1 M Tris-HCl buffer (pH 7.4) containing 1 mM EDTA and 154 mM KCl. The hepatic microsomal fraction was prepared by differential centrifugation (van der Hoeven and Coon, 1974). All procedures of preparation were performed at 0-4°C. Protein content of hepatic microsomes was determined by the method of Lowry et al. (1951), with bovine serum albumin as the standard. About 50 mg of liver tissues were homogenized in 1 ml of TRIzol reagent (Invitrogen, Carlsbad, CA), and total RNA was extracted according to the manufacturer's instructions. The RNA was precipitated using ethanol and stored at -80°C for further analyses.

Leukocytes were isolated from 0.5 ml of blood samples by red blood cell lysis buffer (Roche Diagnostics GmbH Roche Applied Science, Mannheim, Germany), and were suspended either in 0.2 ml of phosphatebuffered saline for DNA extraction or in 1 ml of TRIzol reagent for isolation of total RNA. Genomic DNA was extracted using high pure PCR template preparation kit (Roche Diagnostics GmbH, Mannheim, Germany). Total RNA was isolated from leukocytes similarly to the hepatic RNA extraction. The purity and the concentration of DNA and RNA samples were determined by NanoDrop 1000 spectrophotometer (Thermo Fisher Scientific Inc., Wilmington DE).

In order to determine the effect of the period from liver sampling to RNA extraction, and from blood taking to leukocyte isolation and RNA extraction, *CYP* gene expressions were compared among RNA preparations extracted immediately after liver sampling and blood taking from three donors, and after storage for 4h, 8h and 24h at 4°C. No effect of storage for 4h on CYP mRNA levels was observed; however, some degradation of CYP mRNAs occurred in samples stored for 8h and 24h. Thus, the time of liver tissue and blood delivery, and storage until leukocyte isolation and RNA extraction was limited up to 4h.

**CYP enzyme assays.** Published methods were followed to determine selective enzyme activities: phenacetin O-dealkylation for CYP1A2 (Butler et al., 1989), mephenytoin N-demethylation for CYP2B6 (Heyn et al., 1996), tolbutamide 4-hydroxylation for CYP2C9 (Miners and Birkett, 1996), mephenytoin 4'-hydroxylation for CYP2C19 (Srivastava et al., 1991), dextromethorphan O-demethylation for CYP2D6 (Kronbach et al., 1987), and nifedipine oxidation (Guengerich et al., 1986), midazolam 1'- and 4-hydroxylation (Kronbach et al., 1989) for CYP3A4/5. The incubation mixture contained a NADPH-generating system (1 mM NADPH, 10 mM glucose 6-phosphate, 5 mM MgCl₂ and 2 units/ml glucose 6-phosphate dehydrogenase), human liver microsomes and various substrates selective for CYP isoforms (phenacetin for CYP1A2, tolbutamide for CYP2C9, mephenytoin for CYP2B6 and CYP2C19, dextromethorphan for CYP2D6, or nifedipine and midazolam for CYP3A4/5). The amount of microsomal protein used in enzymatic reactions was 0.8 mg/ml except for phenacetin O-dealkylation (1 mg/ml). Microsomal CYP enzymes worked linearly in a 10-30 min incubation period. Enzyme reactions were terminated by the addition of ice-cold methanol. HPLC analyses were performed according to published methods (Guengerich et al., 1986; Kronbach et al., 1987 and 1989; Butler et al., 1989; Srivastava et al., 1991; Miners and Birkett, 1996; Heyn et al., 1996). All measurements were performed in duplicate with <5% inter- and intraday precision.

**Quantitative real time-PCR.** RNA (3 µg) was reverse transcribed into single stranded cDNA using iScript cDNA synthesis kit (Bio-Rad Laboratories Inc., Hercules CA), and then real time PCR with human cDNA was performed using FastStart Taq DNA polymerase (LightCycler 480 Probes Master, Roche Diagnostics GmbH, Mannheim, Germany) and UPL probes for CYP1A2, CYP2B6, CYP2C9, CYP2C19, CYP2D6 and CYP3A4 (Roche Diagnostics GmbH, Mannheim, Germany). The sequences of primers and probes used for real time-PCR analyses are shown in Table 2. The quantity of target RNA relative to that of housekeeping gene glyceraldehyde 3-phosphate dehydrogenase (GAPDH) was determined. CYP mRNA levels were quantified by real time-PCR measurements in the liver tissues and leukocytes from all donors.

**Table 2: Sequences of PCR primers and probes for CYP-phenotyping and CYP-genotyping**

| **Primer** | **Sequence** | **Probe** | **Sequence** |
|---|---|---|---|
| **For CYP-phenotyping** | | | |
| CYP1A2 forward | 5'-ACAACCCTGCCAATCTCAAG-3' | CYP1A2 | FAM-5'-CTGCCTCT-3'-BHQ |
| reverse | 5'-GGGAACAGACTGGGACAATG-3' | | |
| CYP2B6 forward | 5'-AAAGCGGAGTGTGGAGGA-3' | CYP2B6 | FAM-5'-AGGAGGAG-3'-BHQ |
| reverse | 5'-AAGGTGGGGTCCATGAGG-3' | | |
| CYP2C9 forward | 5'-GTGCACGAGGTCCAGAGATAC-3' | CYP2C9 | FAM-5'-CTTCTCCC-3'-BHQ |
| reverse | 5'-CAGGGAAATTAATATGGTTGTGC-3' | | |
| CYP2C19 forward | 5'-TGAAGGTGGAAATTTTAAGAAAAGTAA-3' | CYP2C19 | FAM-5'-CAGCAGGA-3'-BHQ |
| reverse | 5'-CCCTCTCCCACACAAATCC-3' | | |
| CYP2D6 forward | 5'-TTCCTCAGGCTGCTGGAC-3' | CYP2D6 | FAM-5'-AGGAGGAG-3'-BHQ |
| reverse | 5'-CGCTGGGATATGCAGGAG-3' | | |
| CYP3A4 forward | 5'-CATGGACTTTTTAAGAAGCTTGG-3' | CYP3A4 | FAM-5'-CTCTGCCT-3'-BHQ |
| reverse | 5'-TTCCATGTCAAACATACAAAAGC-3' | | |
| GAPDH forward | 5'-AGCCACATCGCTCAGACAC-3' | GAPDH | FAM-5'-TGGGGAAG-3'-BHQ |
| reverse | 5'-GCCCAATACGACCAAATCC-3' | | |
| **For CYP-genotyping** | | | |
| CYP2C9*2 forward | 5'-AGCAATGGAAAGAAATGGAAG-3' | CYP2C9*2 wild | FAM-CTCTTGAACAC**G**GTCCTC-BHQ1 |
| reverse | 5'-TAAGGTCAGTGATATGGAGTAGG-3' | mutant | CalRed610-CTCTTGAACAC**A**GTCCTC-BHQ2 |
| CYP2C9***3 forward | 5'-GCAAGACAGGAGCCACATG-3' | CYP2C9***3 wild | CalFluorGold540-CGAGGTCCAGAGATAC**A**TTGAC-BHQ1 |
| reverse | 5'-AGGAGAAACAAACTTACCTTGG-3' | mutant | Quasar670-CGAGGTCCAGAGATAC**C**TTGAC-BHQ2 |
| CYP2C19***2 forward | 5'-CTTAGATATGCAATAATTTTCCCAC-3' | CYP2C19***2 wild | CalGold540-TGATTATTTCCC**G**GGAACCCATAAC-BHQ1 |
| reverse | 5'-GAAGCAATCAATAAAGTCCCGA-3' | mutant | Quasar670-TGATTATTTCCC**A**GGAACCCATAAC-BHQ2 |
| CYP2C19***3 forward | 5'-AGATCAGCAATTTCTTAACTTGATG-3' | CYP2C19***3 wild | FAM-ACCCCCTG**G**ATCCAGG-BHQ1 |
| reverse | 5'-TGTACTTCAGGGCTTGGTC-3' | mutant | CalRed610-ACCCCCTG**A**ATCCAGG-BHQ2 |
| CYP2D6***3 forward | 5'-TGGCAAGGTCCTACGC-3' | CYP2D6***3 wild | FAM-CAC**A**GGATGACCTGGGACC-BHQ1 |
| reverse | 5'-TCCATCTCTGCCAGGAAG-3' | mutant | CalRed610-CACGGATGACCTGGGACC-BHQ2 |
| CYP2D6***4 forward | 5'-GGCGACCCCTTACC-3' | CYP2D6***4 wild | CalRed610-CCCCA**G**GACGCCC-BHQ2 |
| reverse | 5'-GATCACGTTGCTCACG-3' | mutant | FAM-CCCCA**A**GACGCCC-BHQ1 |
| CYP2D6***6 forward | 5'-TCTCCGTGTCCACCTTG-3' | CYP2D6***6 wild | FAM-GCTGGAGCAG**T**GGGTGAC-BHQ1 |
| reverse | 5'-GCGAAGGCGGCACA-3' | mutant | CalRed610-GCTGGAGCAGGGGTGAC-BHQ2 |
| CYP3A5***3 forward | 5'-GAGAGTGGCATAGGAGATACC-3' | CYP3A5***3 wild | FAM-TTTGTCTTTCA**A**TATCTCTTCCCTGT-BHQ1 |
| reverse | 5'-TGTACGACACACAGCAACC-3' | mutant | CalRed610-TTTGTCTTTCA**G**TATCTCTTCCCTGT-BHQ2 |

| | | | |
|---|---|---|---|
| FAM, CalRed610, CalFluorGold540, Quasar670: fluorescent labelling, BHQ: black hole quencher | | | |

**CYP-genotyping with TaqMan probes.** Hydrolysis SNP (single nucleotide polymorphism) analysis for *CYP2C9*2, CYP2C9*3, CYP2C19*2, CYP2C19*3, CYP2D6*3, CYP2D6*4, CYP2D6*6* and *CYP3A5*3* was performed by PCR with TaqMan probes (BioSearch Technologies Novato, CA) using the CFX96 real-time PCR detection system (Bio-Rad Laboratories Inc.). Allelic discrimination was based on the design of two TaqMan probes, specific for the wild type allele and the mutant allele labelled with different fluorescent tags (FAM, CalFlourGold540, CalRed610 or Quasar670). Primers and probes (Table 2) were designed based on reference SNP sequences in NCBI reference assembly. The real-time PCR was carried out with 80 ng genomic DNA using FastStart Taq DNA polymerase (LightCycler 480 Probes Master, Roche, or iQ Supermix, Bio-Rad Laboratories Inc., Hercules CA). CYP-genotypes were distinguished by post-PCR allelic discrimination plotted the relative fluorescence values for wild type and mutant alleles. The allelic content of each sample was determined by multicomponent algorithm yielding three allelic clusters representing the CYP-genotypic constituent: homozygous wild type, homozygous mutant type and heterozygous genotype. To confirm the results of CYP-genotyping, a sequence analysis was also performed. 100 ng of DNA were amplified using the primers designed for hydrolysis SNP analysis and iQ Supermix.

**Data analysis.** Hepatic CYP enzyme activities were determined individually in each donor, and the frequency distributions of CYP activities were determined for 164 donors. Three metabolizer phenotypes, poor metabolizers, intermediate metabolizers and extensive metabolizers were statistically distinguished by calculating the quartiles of CYP activity distributions. The cut-off values between the three sub-populations were set to the 1st and the 3rd quartiles of the donors. The quartiles were chosen over standard deviation values as CYP activity distributions were skewed.

Gene expression of *CYP1A2, CYP2B6, CYP2C9, CYP2C19, CYP2D6* and *CYP3A4* were also determined both in the liver and peripheral blood leukocytes. Correlation between hepatic CYP activities and relative CYP mRNA levels in liver or in leukocytes was estimated. The donors with homozygous mutant or heterozygous genotypes were excluded from the correlation analysis for all CYPs except for CYP3A4. For the evaluation of CYP3A4 activity-mRNA correlation, the donors with homozygous wild and heterozygous CYP3A5 genotypes were excluded from the analysis. Correlation among CYP activities and CYP gene expression levels was quantified, and correlation coefficients (rₛ) and 95% confidence intervals were calculated using the Spearman approach (GraphPad InStat version 3.05, San Diego, CA). Strong correlation between gene expression and hepatic CYP activities was considered, if the probability value (P) was under 0.0001.

### Example 2: Correlation between CYP enzyme activities and CYP expression in liver tissues

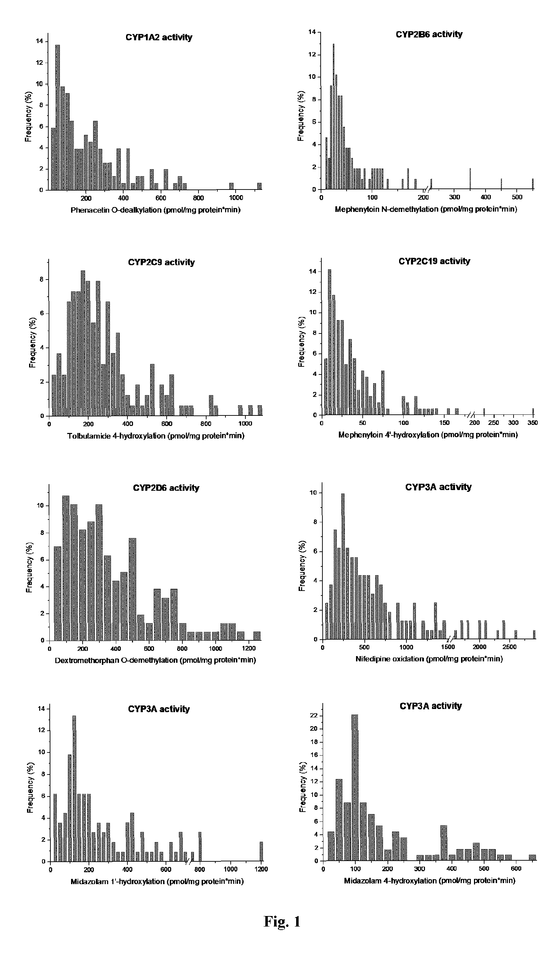

Drug-metabolism in the liver can be roughly estimated by the activities of the most relevant drug-metabolizing CYP enzymes, thus we determined catalytic activities of various CYPs in hepatic microsomal fractions of 164 Hungarian (Caucasian) cadaveric donors. CYP selective substrates that are metabolized by a single CYP isoenzyme to produce a given metabolite were used to measure CYP activities. Figure 1 shows the frequency distributions of CYP1A2, CYP2B6, CYP2C9, CYP2C19, CYP2D6 and CYP3A activities in Hungarian liver donors. The inter-individual variations of CYP activities towards selective marker substrates were wide (80-, 750-fold variation); in several cases, the activities ranged from non-quantifiable to a rather high value (Table 3). CYP activity values did not show Gaussian distribution, but skewed distribution; thus, median±QD values were calculated. The 1st and 3rd quartiles were considered to be the cut-off values between poor and intermediate metabolizers or between intermediate and extensive metabolizers.

**Table 3: Characterization of human liver tissues (n=164) for selective substrates of CYP enzymes. The values are expressed as pmol product/mg microsomal protein*min.**

| **CYP** | **Enzyme activity** | **Min.** | **Max.** | **Cut-off values** | |
|---|---|---|---|---|---|
| | median±QD | | | **PM-IM** | **IM-EM** |
| | | | | 1st quartile | 3 rd quartile |
| CYP1A2 phenacetin O-dealkylation | 155.9±110.75 | 0 | 1107.1 | 60.2 | 281.7 |
| CYP2B6 Mephenytoin N-demethylation | 35.7±22.20 | 6.47 | 538.3 | 22.3 | 66.7 |
| CYP2C9 tolbutamide 4-hydroxylation | 218.2±100.55 | 0 | 1056.0 | 104.4 | 305.6 |
| CYP2C19 mephenytoin 4'-hydroxylation | 25.2±19.15 | 0 | 342.9 | 11.9 | 50.2 |
| CYP2D6 Dextromethorphan O-demethylation | 261.0±162.0 | 0 | 1461.0 | 140.6 | 464.6 |
| CYP3A4/5 nifedipine oxidation | 414.2±272.25 | 3.79 | 2861.5 | 231.8 | 776.3 |
| midazolam 4-hydroxylation | 175.2±141.25 | 11.9 | 1180.0 | 101.1 | 383.6 |
| midazolam 1'-hydroxylation | 107.7±77.4 | 3.42 | 630.7 | 72.8 | 227.6 |

Assays of CYP enzyme activities require a large amount of liver tissues, which can be considered to be a significant drawback in testing drug-metabolizing capacity from liver needle-biopsies, where the available tissue is limited. Real-time PCR techniques can measure CYP expressions in small liver samples. These techniques may provide a useful method for characterization of liver drug-metabolizing capacity, if the hepatic CYP mRNA levels reflect hepatic CYP activities. Total RNA was isolated, and the expression levels of *CYP1A2*, *CYP2B6*, *CYP2C9*, *CYP2C19*, *CYP2D6* and *CYP3A4* genes were determined in the same liver samples in which CYP activities were measured. The primers for each CYP mRNA assay were designed in two consecutive exons separated by an intron on the corresponding genomic DNA; thus the primer pairs amplified cDNA generated from CYP mRNA, and did not yield products on any possible contamination with genomic DNA. All CYP mRNA species investigated were detectable in liver tissues, but at varying levels (Table 4). CYP3A4 mRNA displayed the highest inter-individual variation with the three-magnitude difference between the highest and lowest levels of hepatic expression; whereas CYP2D6 expression exhibited the lowest variation (20-fold) between donors.

**Table 4: CYP mRNA levels relative to GADPH mRNA in liver tissues and in leukocytes of the donors. The values are the mRNA ratio *10⁻³**

| **CYP** | **CYP mRNA levels** | **Min.** | **Max.** |
|---|---|---|---|
| | median±QD | | |
| CYP1A2 - liver | 89.2±55.15 | 3.92 | 678.3 |
| leukocytes | 0.419±0.5026 | 0.0168 | 4.78 |
| CYP2B6 - liver | 57.91±42.80 | 10.9 | 607.1 |
| leukocytes | 3.26±4.643 | 0.00454 | 166.1 |
| CYP2C9-liver | 453.8±237.32 | 30.4 | 1853.2 |
| leukocytes | 0.0178±0.01031 | 0.00022 | 0.0982 |
| CYP2C19 - liver | 179.9±130.14 | 10.17 | 1635.8 |
| leukocytes | 0.0057±0.0056 | 0.000048 | 0.0761 |
| CYP2D6 - liver | 219.7±132.86 | 67.92 | 1274.6 |
| leukocytes | 5.68±40.63 | 0.148 | 16056 |
| CYP3A4-liver | 484.7±406.8 | 2.31 | 4316.9 |
| leukocytes | 0.0362±0.0184 | 0.000009 | 0.218 |

A relationship between hepatic CYP mRNA levels and enzyme activities has been reported by other investigators (Sumida et al., 1999; Rodriguez-Antona et al., 2001). Correlation between CYP selective activities and CYP gene expressions both determined in liver samples of Hungarian donors (n=1 17-124) was estimated. Although the expression of various CYPs would theoretically reflect the drug-metabolizing capacity of the liver, genetic polymorphism of CYPs can give rise perpetually reduced or even ultra-fast metabolism. Several SNPs frequently occur in Caucasian population were determined in donors in parallel with CYP-phenotyping by CYP activities and CYP mRNA levels.

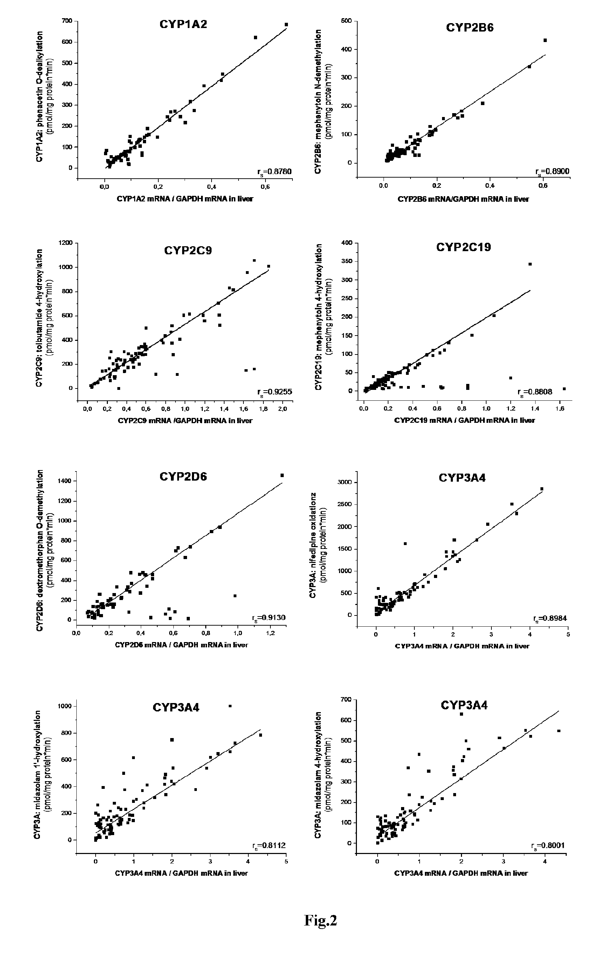

The hepatic CYP1A2 and CYP2B6 mRNA levels correlated significantly with the activities of phenacetin O-dealkylation and mephenytoin N-demethylation, respectively (Fig. 2 and Table 5).

**Table 5: Relationship between CYP enzyme activities and CYP mRNA levels in liver and in leukocytes. Spearman's correlation coefficients (rₛ), 95% confidence intervals (95% CI) and probability values (P) are calculated.**

| **CYP activity** | | **CYP mRNA** | |
|---|---|---|---|
| | | **in liver** | **in leukocytes** |
| CYP1A2 | | CYP1A2 mRNA | |
| phenacetin O-dealkylation | rₛ | 0.8780 | 0.8896 |
| | 95% CI | 0.8058-0.9245 | 0.7868-0.9443 |
| | P | < 0.0001 | < 0.0001 |
| CYP2B6 | | CYP2B6 mRNA | |
| mephenytoin | rₛ | 0.8900 | -0.1824 |
| N-demethylation | 95% CI | 0.8342-0.9278 | -04178-0.07595 |
| | P | < 0.0001 | 0.1525 |
| CYP2C9 | | CYP2C9 mRNA | |
| tolbutamide 4-hydroxylation | rₛ | 0.9255 | 0.9179 |
| | 95% CI | 0.8858-0.9518 | 0.8703-0.9485 |
| | P | < 0.0001 | < 0.0001 |
| CYP2C19 | | CYP2C19 mRNA | |
| mephenytoin | rₛ | 0.8808 | 0.7918 |
| 4'-hydroxylation | 95% CI | 0.8160-0.9237 | 0.6792-0.8679 |
| | P | < 0.0001 | < 0.0001 |
| CYP2D6 | | CYP2D6 mRNA | |
| dextromethorphan | rₛ | 0.9130 | 0.07087 |
| O-demethylation | 95% CI | 0.8532-0.9491 | -0.1939-0.3260 |
| | P | < 0.0001 | 0.5905 |
| CYP3A | | CYP3A4 mRNA | |
| nifedipine oxidation | rₛ | 0.8984 | 0.9475 |
| | 95% CI | 0.8436-0.9346 | 0.9145-0.9680 |
| | P | < 0.0001 | < 0.0001 |
| CYP3A | | CYP3A4 mRNA | |
| midazolam 1'-hydroxylation | rₛ | 0.8112 | 0.7718 |
| | 95% CI | 0.7165-0.8765 | 0.6484-0.8557 |
| | P | < 0.0001 | < 0.0001 |
| CYP3A | | CYP3A4 mRNA | |
| midazolam 4-hydroxylation | rₛ | 0.8001 | 0.8078 |
| | 95% CI | 0.7005-0.8783 | 0.7005-0.8794 |
| | P | < 0.0001 | < 0.0001 |

Somewhat weaker correlation was found between the activities and mRNA expressions of CYP2C9, CYP2C19, CYP2D6 and CYP3A4, if all donors were included in correlation analysis irrespective of their CYP-genotypes (results of the analysis are not shown). *CYP2C9***2* (430C>T) and *CYP2C9***3* (1075A>C) alleles, containing the most common polymorphisms of *CYP2C9* gene produce enzymes with reduced function. The prevalence of the allelic variants for *CYP2C9* was found to be 7.9% for *CYP2C9*2* and 7.0% for *CYP2C9***3.* The donors with mutated *CYP2C9* gene were excluded from correlation analysis, and homozygous wild types were analyzed. Strong association between the hepatic CYP2C9 mRNA levels and tolbutamide 4-hydroxylation activity (rₛ=0.9255) was displayed in donors with *CYP2C9***1*/**1* genotype (Fig. 2 and Table 5). Mutations in *CYP2C19* gene, resulting in non-functional *CYP2C19* alleles, *CYP2C19***2* (681G>A) and *CYP2C19***3* (636G>A) were also determined in liver donors. *CYP2C19***2* allelic variant was detected with the frequency of 18.3% in the liver donors, whereas *CYP2C19***3* allele was not observed at all. Excluding the donors carrying *CYP2C19***2* allele from correlation analysis, the hepatic mephenytoin 4-hydroxylation activity of the donors with *CYP2C19***1*/**1* genotype significantly correlated with CYP2C19 mRNA levels in the liver. Deficiency of *CYP2D6* gene, resulting in *CYP2D6***3* (2549de1A) and *CYP2D6***4* (1846G>A) alleles is associated with the lack of enzyme activity, whereas *CYP2D6***6* mutation (1795delT) leads to the lack of enzyme protein and consequently to the lack of CYP2D6 activity. The prevalence of *CYP2D6***4* allelic variant was relatively high 17.4%, while the occurrence of both *CYP2D6***3* and *CYP2D6***6* was found to be 0.89%. Correlation analysis was carried out with the donors having exclusively wild type *CYP2D6* gene, and strong association was found between the hepatic CYP2D6 mRNA levels and dextromethorphan O-demethylase activities (rₛ=0.8808).

CYP3A4 forms the bulk of the hepatic CYP3A protein and activity (about 95% of CYP3A pool); however, the other members, primarily CYP3A5 can also contribute to the metabolism of CYP3A substrates in adult liver. *CYP3A5***3* mutation (6986A>G in intron 3) results in splicing defect, leading to the lack of CYP3A5 enzyme. The estimated *CYP3A5***3* allele frequency is more than 90% in Caucasian population. Those individuals who have functional CYP3A5 enzyme (with *CYP3A5***1*/**1* and *CYP3A5***1*/**3* genotypes) metabolize some CYP3A substrates (e.g. tacrolimus, cyclosporine A, nifedipine, midazolam) more rapidly than CYP3A5 non-expressors. 89.5% of Hungarian donors did not express functional CYP3A5, carrying *CYP3A5***3*/**3* genotype. We did not find homozygous wild genotype (*CYP3A5***1*/**1*) among the donors investigated; thus, functional *CYP3A5***1* allele was detected only in donors with heterozygous genotype. The frequency of wild type (*CYP3A5***1*) allele was found to be 5.5% in liver donors. Excluding the donors with *CYP3A5***1*/**3* genotype from correlation analysis, strong correlation was displayed between the hepatic CYP3A4 expression and all three CYP3A activities, nifedipine oxidation, midazolam 1'- and 4-hydroxylation (Fig. 2 and Table 5).

### Example 3: Correlation between CYP enzyme activities in liver and CYP expression in leukocytes

Liver biopsies are generally not available from patients; however, information on drug-metabolizing capacity obtained from leukocytes would be of clinical interest, if CYP mRNA levels in leukocytes reflect the hepatic CYP activities. Leukocytes were isolated from blood samples, and the expression levels of *CYP* genes in leukocytes were determined in the same donors whose hepatic CYP activities and CYP mRNA levels were measured. The CYP expression profiles of the leukocytes showed some similarities to the liver; however, the expression levels displayed significant differences. All CYP mRNAs that were detected in liver tissues were also expressed in leukocytes. Relative expression of various CYPs was generally 10^{2 _} 10⁴ fold higher in liver tissues than in leukocytes except for CYP1A2 and CYP2B6 (Table 4). The expression of *CYP1A2* and *CYP2B6* genes in leukocytes was merely 200- and 20-fold lower, respectively than in liver tissues. High inter-individual variations of CYP expression were also detected in leukocytes similarly to the liver tissues. The largest variations (10^{4 _} 10⁵ fold) between individuals were found in the expression of CYP2B6, CYP2D6 and CYP3A4 in leukocytes, whereas CYP1A2 and CYP2C9 mRNA levels displayed only 285- and 445-fold variations, respectively.

Taking blood from patients and isolating leukocytes is a simple way to obtain biological material that may be assumed to provide sufficient information on hepatic drug-metabolizing capacity. Our aim was to study the relationship between CYP enzyme activities in liver and the expression of *CYP* genes in leukocytes collected simultaneously. If Spearman's correlation coefficient was higher than 0.7, we defined CYP expression levels in leukocytes and the corresponding hepatic CYP activities as closely associated.

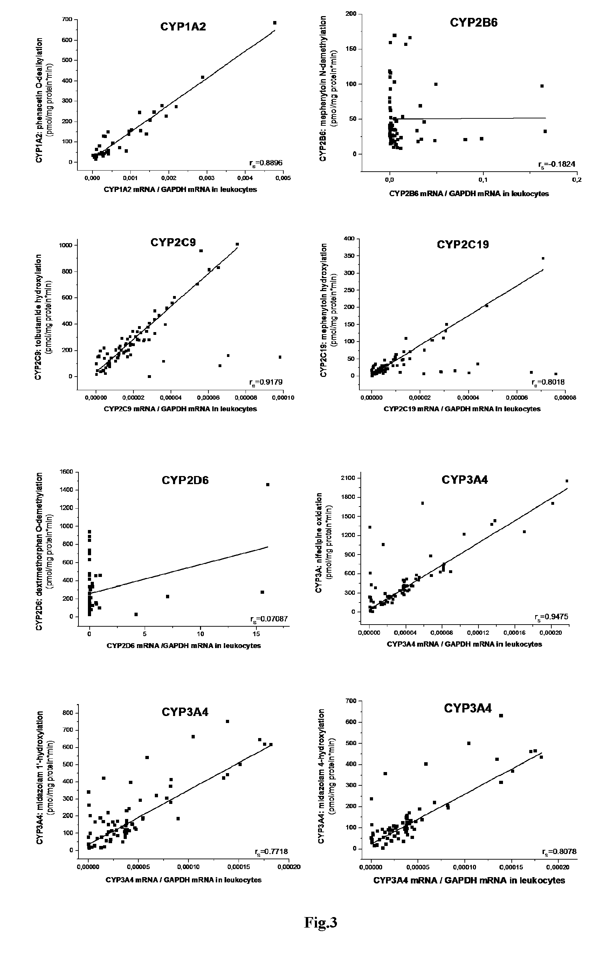

The CYP1A2 mRNA levels in leukocytes correlated significantly with the activity of phenacetin O-dealkylation in liver (Fig. 3, Table 5). Thus we can conclude that CYP1A2 expression in leukocytes reflects the hepatic CYP1A2 activity. The donors carrying mutated *CYP2C9* alleles (*CYP2C9***2* or *CYP2C9***3*) were excluded from correlation analysis. Strong association between CYP2C9 mRNA levels in leukocytes and tolbutamide 4-hydroxylation activity in liver was also displayed in donors with *CYP2C9***1*/**1* genotype. The expression levels of CYP2C19 in leukocytes were closely associated with mephenytoin 4-hydroxylation in liver of the donors carrying *CYP2C19***1*/**1* genotype. In conclusion, the leukocytes isolated from subjects, carrying wild type *CYP2C9* or *CYP2C19,* reflect hepatic CYP2C9 and CYP2C19 activities. Furthermore, all three CYP3A activities in hepatic microsomes displayed close correlation with CYP3A4 mRNA levels in leukocytes, if we excluded donors with *CYP3A5***1* alleles from correlation analysis. On the other hand, no association could be observed for the expression of CYP2B6 or CYP2D6 in leukocytes and hepatic enzyme activities, mephenytoin N-demethylation and dextromethorphan O-demethylation, respectively (rₛ=-0.1824, P=0.1525 for CYP2B6 and rₛ=0.07087, P=0.5905 for CYP2D6).

### Example 4: Determining cut-off values for distinguishing poor, intermediate and extensive metabolizers

The drug-metabolizing capacity of the liver was suggested to qualify according to the frequency distributions of hepatic CYP1A2, CYP2B6, CYP2C9, CYP2C19, CYP2D6 and CYP3A4 activities in 164 Hungarian donors. The 1st and 3rd quartiles determined the cut-off values between poor and intermediate metabolizers or between intermediate and extensive metabolizers (Table 3). The expression of these CYPs in liver displayed strong correlation with hepatic CYP activities; therefore the estimation of a patient's drug-metabolizing capacity can be carried out on the basis of mRNA levels in liver biopsy sample. The optimal cut-off values for mRNAs levels of CYP1A2, CYP2B6, CYP2C9, CYP2C19, CYP2D6 and CYP3A4 in liver were set on the basis of the cut-off values for hepatic CYP activities, allowing of distinguishing poor, intermediate and extensive metabolizers (Table 6). The mRNA levels of CYP2B6 and CYP2D6 in leukocytes did not correlate with the respective CYP activities in liver, and consequently leukocytes cannot serve as appropriate cells for the assessment of hepatic CYP2B6 and CYP2D6 activities. However, the expression of CYP1A2, CYP2C9, CYP2C19 and CYP3A4 in leukocytes was proven to reflect to the respective CYP activities in liver, thus the hepatic activities of these CYP enzymes were suggested to be qualified by leukocyte mRNA levels of these CYP species. The cut-off values for mRNAs levels of CYP1A2, CYP2C9, CYP2C19 and CYP3A4 in leukocytes were also established on the basis of the cut-off values for hepatic CYP activities, allowing of distinguishing poor, intermediate and extensive metabolizers (Table 6).

**Table 6: Cut-off values for CYP mRNA levels relative to GADPH mRNA in liver tissues and in leukocytes distinguishing poor (PM), intermediate (IM) and extensive (EM) metabolizers.**

| **CYP** | **Cut-off values** | |
|---|---|---|
| | **PM-IM** | **IM-EM** |
| CYP1A2-liver | 4.5*10⁻² | 1.5*10⁻¹ |
| leukocytes | 10⁻⁴ | 2*10⁻³ |
| CYP2B6-liver | 3.5*10⁻² | 1.5*10⁻¹ |
| leukocytes | - | - |
| CYP2C9-liver | 1.5*10⁻¹ | 7*10⁻¹ |
| leukocytes | 5*10⁻⁶ | 2.5*10⁻⁵ |
| CYP2C19-liver | 8*10⁻² | 3.5*10⁻¹ |
| leukocytes | 10⁻⁶ | 10⁻⁵ |
| CYP2D6-liver | 1.5*10⁻¹ | 5*10⁻¹ |
| leukocytes | - | - |
| CYP3A4-liver | 2.5*10⁻¹ | 1.5 |
| leukocytes | 10⁻⁶ | 10⁻⁴ |

CYPtest™ investigations indicating reduced (or extensive) drug-metabolizing capacity of patients and rationalization of drug therapy resulted in successful outcomes.

### Example 4: Case reports

1. 11-year old girl suffered from congenital liver fibrosis (liver disease leading to fibrotic degeneration) underwent liver transplantation. The postoperative course was uneventful and showed normal graft function; however, signs of rejection occurred two weeks after transplantation. Immunological reactions and infections were excluded as the reason of rejection. Testing drug-metabolizing capacity of the donor liver indicated extremely reduced CYP2C9 activity. This enzyme catalyzes the metabolism of the antifungal Mycosyst (fluconazole), the antibacterial Sumetrolim (sulfamethoxazole, trimethoprim) and the Losec (omeprazol) used in ulcus prevention therapy. The results of histopathological examination also confirmed potential drug toxicity. Withdrawal of these drugs was suggested because of functional CYP2C9 deficiency of the graft. In consequence of alterations in medication, the liver graft recovered within one week, and the patient left the hospital. Currently, her days are similar to the lives of other healthy children.
2. The only option for a 17-year-old girl with primary sclerosing cholangitis leading to cancerous processes was liver transplantation. One week after transplantation, significant increase in levels of transaminases, alkaline phosphatase or bilirubin indicated liver function failure. In the test of drug-metabolizing status, the liver graft showed strongly reduced activity of CYP2C19. Rationalization of medication, - withdrawal of Mycosyst (fluconazole) and replacement of Controloc (pantoprazole) to Quamatel (famotidine), - was proposed for the recipient. CYP2C19 is not involved in famotidine metabolism. In consequence of alterations in medication, the patient recovered within one week, and left the clinic after two weeks.
3. A 1.5-month old new-born suffered from epilepsy was treated with valproic acid; however, some toxic symptoms were observed after two weeks (anemia, thrombocytopenia). CYPtest^{™} demonstrated genetic defects in *CYP2C9* gene (*CYP2C9***3*/**3*) which results non-functional CYP2C9 enzyme. CYP2C9 is responsible for the metabolism of valproic acid. CYP-genotyping results were also confirmed by high blood level of valproic acid which led to toxic symptoms. On the basis of CYPtest^{™} results, the drug was withdrawn and replaced with an alternative drug (carbamazepine). The patient's status is stable and her basic disease is successfully controlled by carbamazepine.
4. Gestation hypertension developed at the 21st gestation week of a 35-year old pregnant woman who was treated with methyl-dopa (Dopegyt). On the 23rd gestation week acute hepatitis developed, thus the antihypertensive therapy was changed from methyl-dopa to nifedipine. CYP3A4 enzyme plays a primary role in the metabolism of nifedipine. CYPtest^{™} indicated poor CYP3A4 metabolizing capacity, therefore reduced nifedipine dose (30 mg daily) was proposed. Blood pressure was successfully controlled with reduced nifedipine therapy. Throughout the gestation period, CYP3A4 expression was followed and nifedipine dosage was adjusted to CYP3A4 status. Finally, a healthy baby was born on the 40th gestation week.
5. CYPtesting a 33-year old woman with schizoid disorder, we demonstrated *CYP3A5***1*/**3* heterozygous genotype which produces functional CYP3A5 enzyme protein. Functional CYP3A5 enzyme is lacking in more than 90% of Hungarian population which means that most of the population has *CYP3A5***3*/**3* genotype. Production of functional CYP3A5 enzyme (*CYP3A5***1*/**3*) leads to an increase in CYP3A activity, since the activities of CYP3A5 and CYP3A4 are summed resulting in faster metabolism of certain drugs. Dose increase of these drugs would be required for efficient therapy. The patient in our case was treated with carbamazepine in a daily dose of 400 mg. Since carbamazepine is primarily metabolized by CYP3A enzymes, carbamazepine level in patient's blood was lower than the therapeutic concentration. Dose increase was proposed to 800 mg which resulted in less pronounced emotional fluctuation, stabilized mood and more balanced lifestyle.

### References

Blanco JG, Harrison PL, Evans WE, and Relling MV (2000) Human cytochrome P450 maximal activities in pediatric versus adult liver. Drug Metab Dispos 28: 379-382.
Brockmöller J, Kirchheiner J, Meisel C, and Roots I (2000) Pharmacogenetic diagnostics of cytochrome P450 polymorphisms in clinical drug development and in drug treatment. Pharmacogenomics 1: 125-151.
Butler MA, Iwasaki M, Guengerich FP, and Kadlubar FF (1989) Human cytochrome P-450pA (P-450IA2), the phenacetin O-deethylase, is primarily responsible for the hepatic 3-demethylation of caffeine and N-oxidation of carcinogenic arylamines. Proc Natl Acad Sci USA 86: 7696-7700.
Chen Q, Zhang T, Wang JF, and Wie DQ (2011) Advances in human cytochrome P450 and personalized medicine. Curr Drug Metab 12: 436-444.
Dai Y, Hebert MF, Isoherranen N, Davis CL, Marsh C, Shen DD, and Thummel KE (2006) Effect of CYP3A5 polymorphism on tacrolimus metabolic clearance in vitro. Drug Metab Dispos 34: 836-847.
Finnström N, Thörn M, Lööf L, and Rane A (2001) Independent patterns of cytochrome P450 gene expression in liver and blood in patients with suspected liver disease. Eur J Clin Pharmacol 57: 403-409.
Finnström N, Ask B, Dahl M-L, Gadd M, and Rane A (2002) Intra-individual variation and sex differences in gene expression of cytochromes P450 in circulating leukocytes. Pharmacogenomics J 2: 111-116.
Furukawa M, Nishimura M, Ogino D, Chiba R, Ikai I, Ueda N, Naito S, Kuribayashi S, Moustafa MA, Uchida T, Sawada H, Kamataki T, Funae Y, and Fukumoto M (2004) Cytochrome P450 gene expression levels in peripheral blood mononuclear cells in comparison with the liver.Cancer Sci 95: 520-529.
George J, Liddle C, Murray M, Byth K, and Farrell GC (1995) Pre-translational regulation of cytochrome P450 genes is responsible for disease-specific changes of individual P450 enzymes among patients with cirrhosis. Biochem Pharmacol 49: 873-881.
Guengerich FP, Martin MV, Beaune PH, Kremers P, Wolff T, and Waxman DJ (1986) Characterization of rat and human liver microsomal cytochrome P-450 forms involved in nifedipine oxidation, a prototype for genetic polymorphism in oxidative drug metabolism. J Biol Chem 261: 5051-5060.
Heyn H, White RB, and Stevens JC (1996) Catalytic role of cytochrome P4502B6 in the N-demethylation of S-mephenytoin. Drug Metab Dispos 24: 948-954.
van der Hoeven TA, and Coon MJ (1974) Preparation and properties of partially purified cytochrome P-450 and reduced nicotinamide adenine dinucleotide phosphate-cytochrome P-450 reductase from rabbit liver microsomes. J Biol Chem 249: 6302-6310.
Huang W, Lin YS, McConn DJ, Calamia JC, Totah RA, Isoherranen N, Glodowski M, and Thummel KE (2004) Evidence of significant contribution from CYP3A5 to hepatic drug metabolism. Drug Metab Dispos 32: 1434-1445.
Ingelman-Sundberg M (2001) Pharmacogenetics: an opportunity for a safer and more efficient pharmacotherapy. J Intern Med 250: 186-200.
Kabanova S, Kleinbongard P, Volkmer J, Andree B, Kelm M, and Jax TW (2009) Gene expression analysis of human red blood cells. Int J Med Sci 6: 156-159.
Koch I, Weil R, Wolbold R, Brockmöller J, Hustert E, Burk O, Nuessler A, Neuhaus P, Eichelbaum M, Zanger U, and Wojnowski L (2002) Interindividual variability and tissue-specificity in the expression of cytochrome P450 3A mRNA. Drug Metab Dispos 30: 1108-1114.
Kronbach T, Mathys D, Gut J, Catin T, and Meyer UA (1987): High-performance liquid chromatographic assays for bufuralol 1'-hydroxylase, debrisoquine 4-hydroxylase, and dextromethorphan O-demethylase in microsomes and purified cytochrome P-450 isozymes of human liver. Anal Biochem 162: 24-32.
Kronbach T, Mathys D, Umeno M, Gonzalez FJ, and Meyer U (1989) Oxidation of midazolam and triazolam by human liver cytochrome P450IIIA4. Mol Pharmacol 36: 89-96.
Lee CY, Lai TY, Wu YM, Hu RH, Lee PH, Hsu LC, and Tsai MK (2010) Gene expression of P-glycoprotein and cytochrome P450 3A4 in peripheral blood mononuclear cells and correlation with expression in liver. Transplant Proc 42: 834-836.
Lewis DFV (2004) 57 varieties: the human cytochromes P450. Pharmacogenomics 5: 305-318.
Lind A-B, Wadelius M, Darj E, Finnström N, Lundgren S, and Rane A (2003) Gene expression of cytochrome P450 IB1 and 2D6 in leukocytes in human pregnancy. Pharmacol Toxicol 92: 295-299.
Lowry OH, Rosebrough NJ, Farr AL, and Randall RJ (1951) Protein measurement with Folin phenol reagent. J Biol Chem 193: 265-275.
Miners JO, and Birkett DJ (1996) Use of tolbutamide as a substrate probe for human hepatic cytochrome P450 2C9. Methods in Enzymol 272: 139-145.
Monostory K, and Pascussi J-M (2008) Regulation of drug-metabolizing human cytochrome P450s. Acta Chim Slo 55: 20-37.
Niwa T, Shiraga T, Yamasaki S, Ishibashi K, Ohno Y, and Kagayama A (2003) In vitro activation of 7-benzyloxyresorufin O-debenzylation and nifedipine oxidation in human liver microsomes. Xenobiotica 33: 717-729.
Nowakowski-Gashaw I, Mrozikiewicz PM, Roots I, and Brockmöller J (2002) Rapid quantification of CYP3A4 expression in human leukocytes by real-time reverse transcription-PCR. Clin Chem 48: 366-370.
Patki KC, von Moltke LL, and Greenblatt DJ (2003) In vitro metabolism of midazolam, triazolam, nifedipine, and testosterone by human liver microsomes and recombinant cytochromes p450: role of CYP3A4 and CYP3A5. Drug Metab Dispos 31: 938-944.
Rodriguez-Antona C, Donato MT, Pareja E, Gomez-Lechon MJ, and Castell JV (2001) Cytochrome P-450 mRNA expression in human liver and its relationship with enzyme activity. Arch Biochem Biophys 393: 308-315.
Shu Y, Cheng Z-N, Liu Z-Q, Wang L-S, Zhu B, Huang S-L, Ou-Yang D-S, and Zhou H-H (2001) Interindividual variations in levels and activities of cytochrome P-450 in liver microsomes of Chinese subjects. Acta Pharmacol Sin 22: 283-288.
Solus JF, Arietta BJ, Harris JR, Sexton DP, Steward JQ, McMunn C, Ihrie P, Mehall JM, and Edwards EP (2004) Genetic variation in eleven phase I drug metabolism genes in an ethnically diverse population. Pharmacogenomics 5: 895-931.
Srivastava PK, Yun C-H, Beaune PH, Ged C, and Guengerich FP (1991) Separation of human liver microsomal tolbutamide hydroxylase and (S)-mephenytoin 4'-hydroxylase cytochrome P-450 enzymes. Mol Pharmacol 40: 69-79.
Sumida A, Kinoshita K, Fukuda T, Matsuda H, Yamamoto I, Inaba T, and Azuma J (1999) Relationship between mRNA levels quantified by reverse transcription-competitive PCR and metabolic activity of CYP3A4 and CYP2E1 in human liver. Biochem Biophys Res Commun 262: 499-503.
Thörn M, Lundgren S, Herlenius G, Ericzon BG, Lööf L, and Rane A (2004) Gene expression of cytochromes P450 in liver transplants over time. Eur J Clin Pharmacol 60: 413-420.
Transon C, Lecoeur S, Leemann T, Beaunne P, and Dayer P (1996) Interindividual variability in catalytic activity and immunreactivity of three major human liver cytochrome P450 isozymes. Eur J Clin Pharmacol 51: 79-85.
Wilke RA, Musana AK, and Weber WW (2005) Cytochrome P450 gene-based drug prescribing and factors impacting translation into routine clinical practice. Person Med 2: 213-224.
Yuan R, Madani S, Wei XX, Reynolds K, and Huang SM (2002) Evaluation of cytochrome P450 probe substrates commonly used by the pharmaceutical industry to study in vitro drug interactions. Drug Metab Dispos 30: 1311-1319.

## Claims

1. Method for the estimation of a patient's drug-metabolizing capacity by parallel CYP (cytochrome P 450) -genotyping and CYP-phenotyping of said patient, comprising
a) identification of specific mutation(s) within the gene encoding said CYP enzyme from a leukocyte sample obtained from said patient,
b) determination of the expression level of the gene encoding said CYP enzyme from said leukocyte sample,
c) estimation of the drug-metabolizing capacity of said patient on the basis of CYP-genotyping data and pre-established cut-off values of said expression level determined for said leukocyte sample, thereby qualifying said patient as poor, intermediate or extensive metabolizer.

2. The method according to claim 1, wherein said CYP enzyme is the CYP1A2, CYP2C9, CYP2C19 and/or CYP3A4.

3. The method according to any one of claims 1 to 2, wherein said determination of specific mutations within the gene encoding said CYP enzyme is made by single nucleotide polymorphism analysis.

4. The method according to any one of claims 1 to 3, wherein said determination of the expression level of the gene encoding said CYP enzyme is made by real time-PCR analysis.
